# FASCICULE DE BREVET EUROPEEN

(11) **EP 3 615 036 B1**
(45) Date de publication et mention de la délivrance du brevet: **22.11.2023**
(21) Numéro de dépôt: 18721042.2
(22) Date de dépôt: 30.04.2018
(51) Int. Cl.: A61K 31/575, A61K 36/28, A61P 21/00

(54) **PRÉPARATION D'UN EXTRAIT DE 20-HYDROXYECDYSONE DE QUALITÉ PHARMACEUTIQUE**
HERSTELLUNG VON 20-HYDROXYEKDYSONEXTRACT IN PHARMAZEUTISCHER QUALITÄT
PREPARATION OF A PHARMACEUTICAL GRADE 20-HYDROXYECDYSONE EXTRACT

(30) Priorité: 28.04.2017 FR 1753775; 31.08.2017 FR 1758071
(43) Date de publication de la demande: 04.03.2020
(73) Titulaire: Biophytis, 75001 Paris (FR); Sorbonne Université, 75006 Paris (FR)
(72) Inventeur: LAFONT, René, 75016 Paris (FR); DILDA, Pierre, 75016 Paris (FR); DIOH, Waly, 91220 Bretigny Sur Orge (FR); DUPONT, Philippe, 75013 Paris (FR); DEL SIGNORE, Susanna, 92100 Boulogne Billancourt (FR); VEILLET, Stanislas, 91600 Savigny Sur Orge (FR)
(74) Mandataire: Ipside
(86) Numéro de dépôt international: PCT/EP2018/061060
(87) Numéro de publication internationale: WO 2018/197731

(56) Documents cités:
- Anonymous: "Biophytis presents preliminary clinical data of SARA-PK, and new preclinical data of Sarconeos for treating sarcopenia", , 16 décembre 2016 (2016-12-16), pages 1-4, XP055430042, Extrait de l'Internet: URL:http://www.biophytis.com/wp-content/up loads/2015/05/PR-BIOPHYTIS-Posters-SCWD-16 1212_ENG-.pdf [extrait le 2017-11-29]
- Anonymous: "Biophytis", , 24 septembre 2015 (2015-09-24), pages 1-32, XP002778985, Extrait de l'Internet: URL:http://www.biophytis.com/wp-content/up loads/2015/09/INVEST-SECURITIES-BIOPHYTIS- REPORT-092215-EN.pdf [extrait le 2018-03-08]
- Administrator: "Abstracts of the 9th International Conference on Cachexia, Sarcopenia and Muscle Wasting, Berlin, Germany, 10-11 December 2016 (part 1)", JCSM - Journal of Cachexia, Sarcopenia and Muscle, 18 novembre 2016 (2016-11-18), pages 1-22, XP55430368, Extrait de l'Internet: URL:http://www.jcsm.info/index.php/en/comp onent/content/article/146-abstracts-volume -7/v7-number5-december-2016/856-a-systemat ic-review-on-the-role-of-vitamins-minerals -proteins-and-other-supplements-for-the-tr eatment-of-cachexia-in-cancer-a-european-p alliative-care-research-centre-cachexia-pr oject-2 [extrait le 2017-11-29]
- TOTH N ET AL: "20-Hydroxyecdysone increases fiber size in a muscle-specific fashion in rat", PHYTOMEDICINE, ELSEVIER, AMSTERDAM, NL, vol. 15, no. 9, 3 septembre 2008 (2008-09-03), pages 691-698, XP023612056, ISSN: 0944-7113, DOI: 10.1016/J.PHYMED.2008.04.015 [extrait le 2008-06-26]
- NOEMI TOTH ET AL: "Preparative-Scale Chromatography of Ecdysteroids: A Class of Biologically Active Steroids", JOURNAL OF CHROMATOGRAPHIC SCIENCE, 15 février 2008 (2008-02-15), pages 111-116, XP055430432,
- GALLO M B C ET AL: "Quantitative determination of 20-hydroxyecdysone in methanolic extract of twigs from Vitex polygama Cham", JOURNAL OF CHROMATOGRAPHY B: BIOMEDICAL SCIENCES & APPLICATIONS, ELSEVIER, AMSTERDAM, NL, vol. 832, no. 1, 17 février 2006 (2006-02-17), pages 36-40, XP027981747, ISSN: 1570-0232 [extrait le 2006-02-17]
- LI-JUN TAN ET AL: "Molecular genetic studies of gene identification for sarcopenia", HUMAN GENETICS, SPRINGER, BERLIN, DE, vol. 131, no. 1, 26 juin 2011 (2011-06-26) , pages 1-31, XP019992518, ISSN: 1432-1203, DOI: 10.1007/S00439-011-1040-7

## Description

### DOMAINE TECHNIQUE DE L'INVENTION

La présente invention concerne la préparation d'extraits de 20-hydroxyecdysone (20E) purifiée qui peuvent être utilisés thérapeutiquement, en particulier pour l'amélioration de la fonction musculaire, en augmentant la masse et la force musculaire ainsi que la mobilité chez les mammifères, la 20E ayant des effets anabolisants chez les mammifères.

Plus particulièrement l'invention permet l'obtention d'extraits permettant d'améliorer et/ou de prévenir la dégradation de la fonction musculaire des mammifères sarcopéniques ou de mammifères ayant été immobilisés notamment après une intervention chirurgicale ou atteints de dystrophies musculaires notamment d'origine génétique.

En outre, l'invention permet l'obtention d'extraits permettant de lutter contre les maladies cardio-métaboliques dont les maladies cardiovasculaires et le diabète, grâce aux effets métaboliques de la 20E.

### ÉTAT DE LA TECHNIQUE

Les phytoecdysones représentent une importante famille de stérols polyhydroxylés. Ces molécules sont produites pas diverses espèces de plantes (fougères, gymnospermes, angiospermes) et participent à la défense de ces plantes contre les ravageurs.

De par leur abondance dans la nature, et la difficulté de leur synthèse chimique, les phytoecdysones proposées dans divers compléments alimentaires sont extraites à partir de plantes cultivées à cette fin. Ces plantes produisent un cocktail complexe de phytoecdysones (e.g. Bathori et al., 1999 ; Kokoska & Jankovska, 2009) et leurs extraits contiennent majoritairement de la 20E, mais aussi un ensemble de phytoecdysones dites mineures, ainsi que des composés appartenant à d'autres familles chimiques, la somme des phytoecdysones représentant selon les préparations de 5 à 90% des extraits.

La nature des phytoecdysones mineures dépend des plantes utilisées comme matière première (Hunyadi et al., 2016), de la saison à laquelle est faite la récolte, voire de la région dans laquelle elles sont cultivées (sol, climat) et est de ce fait susceptible de varier sensiblement d'un lot à un autre. Ces variations sont progressivement atténuées au cours de la purification.

Les données de la littérature décrivent la 20E comme faiblement cytotoxique mais, si cela s'applique à la molécule pure, il n'en va pas de même des préparations incomplètement purifiées.

Dans le but de mettre à profit les effets bénéfiques de la 20E sur la qualité musculaire dans le cadre d'un médicament, il est nécessaire de disposer d'un extrait de 20E de grade pharmaceutique.

Un extrait de 20E au grade pharmaceutique (≥ 97%) à partir d'un extrait purifié à 90% n'est à l'heure actuelle obtenu que par la mise en oeuvre de méthodes chromatographiques (BPLC, HPLC). Ces méthodes, telle celle exposée par Toth et Bathori (2008), sont adaptées pour la production de quantités d'extraits de l'ordre de quelques grammes.

### OBJET DE L'INVENTION

La présente invention vise l'obtention d'un extrait de plante ou d'une partie de plante, ladite plante étant choisie parmi les végétaux contenant au moins 0,5% de 20-hydroxyecdysone en poids sec dudit végétal, ledit extrait comportant au moins 95%, et de préférence au moins 97%, de 20-hydroxyecdysone.

L'extrait obtenu par mise en oeuvre de l'invention, aussi appelé par la suite BIO101, est de façon remarquable dépourvu d'impuretés, comme des composés mineurs, susceptibles d'affecter l'innocuité, la disponibilité ou l'efficacité d'une application pharmaceutique dudit extrait.

Les impuretés absentes de BIO101 sont des composés à 19 ou 21 atomes de carbone, comme la Rubrostérone, la Dihydrorubrostérone ou la Poststérone.

La plante à partir de laquelle est produit BIO101 est avantageusement choisie parmi *Stemmacantha carthamoides* (aussi appelée *Leuzea carthamoides*), *Cyanotis arachnoidea* et *Cyanotis vaga.* L'invention vise plus particulièrement l'obtention de BIO101 à partir des racines de ces plantes.

L'invention s'intéresse tout spécialement à l'obtention d'un extrait de racines de *Stemmacantha carthamoides* comportant au moins 95%, et de préférence au moins 97%, de 20-hydroxyecdysone.

A cet effet, l'invention concerne un procédé de préparation d'un extrait comportant au moins 95%, de préférence au moins 97%, de 20-hydroxyecdysone, à partir d'une préparation de 20-hydroxyecdysone pure de l'ordre de 90%, caractérisé en ce qu'il comprend les étapes suivantes :
a) Dissolution à chaud de 20-hydroxyecdysone pure de l'ordre de 90% dans du méthanol, filtration et concentration partielle,
b) Ajout de 1 à 5 volumes d'acétone,
c) Refroidissement à une température comprise entre 0 et 5°C, sous agitation,
d) Filtration du précipité obtenu,
e) Rinçages successifs avec de l'acétone et de l'eau, et
f) Séchage.

Cette purification met en jeu un processus de recristallisation approprié à cette molécule et apte à être réalisé à l'échelle industrielle.

De manière avantageuse, la filtration de l'étape a) est réalisée grâce à un filtre à particules de 0,2 µm.

La concentration partielle de l'étape a) est avantageusement effectuée par distillation sous vide, à une température de l'ordre de 50°C, en présence de MeOH.

L'étape f) de séchage est effectuée sous vide à une température de l'ordre de 50°C.

Selon un exemple de réalisation, l'étape b) consiste en l'ajout de 3 volumes d'acétone.

Avantageusement, le procédé de préparation d'un extrait objet de l'invention permet d'obtenir un extrait de 20E de grade pharmaceutique. Le procédé de préparation d'un extrait objet de l'invention permet de travailler à l 'échelle industrielle.

Grâce au procédé selon l'invention, la 20E est purifiée au grade pharmaceutique et BIO101 peut être utilisé pour des applications pharmaceutiques diverses.

De manière avantageuse, BIO101 est destiné à être utilisé pour améliorer et/ou prévenir la dégradation de la fonction musculaire de mammifères sarcopéniques ou de mammifères ayant été immobilisés, notamment après une intervention chirurgicale, ou atteints de dystrophies musculaires, notamment d'origine génétique. En outre, l'utilisation de BIO101 permet de lutter contre les maladies cardio-métaboliques dont les maladies cardiovasculaires et le diabète, grâce aux effets métaboliques de la 20E.

BIO101 est destiné à être utilisé dans le traitement d'affections musculaires ou cardio-métaboliques.

### BRÈVE DESCRIPTION DES FIGURES

D'autres avantages, buts et caractéristiques particulières de la présente invention ressortiront de la description non limitative qui suit d'au moins un mode de réalisation particulier des dispositifs objets de la présente invention, en regard des dessins annexés, dans lesquels :
- La figure 1 représente schématiquement la structure de la 20-hydroxyecdysone (20E).
- La figure 2 illustre schématiquement le procédé de purification de la 20E selon l'invention.
- La figure 3 illustre le profil pharmacocinétique de différentes cohortes d'individus ayant reçu une administration unique d'extrait obtenu selon l'invention (BIO101).
- La figure 4 est un diagramme représentatif des effets du traitement chronique de BIO101, sur la vitesse maximale de course de souris âgées.

### DESCRIPTION DÉTAILLÉE DE MODES DE RÉALISATION DE L'INVENTION

La présente description est donnée à titre non limitatif.

### 1. Procédé de purification par recristallisation

Le procédé a été développé en partant d'une préparation de 20E extraite de Stemmacantha carthamoides, mais il peut s'appliquer à des préparations de 20E issues d'autres plantes.

Une fiche technique de 20E pure à 90% utilisable comme préparation initiale est décrite en annexe.

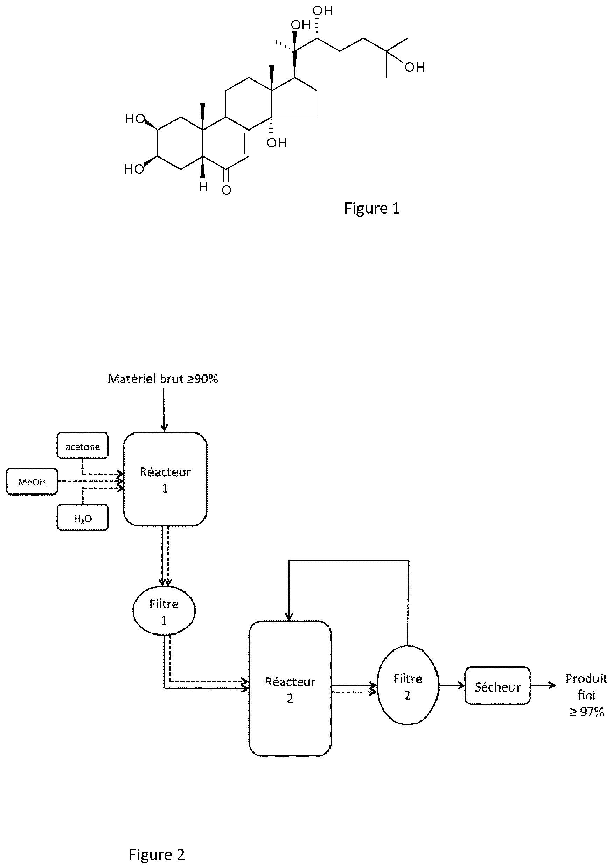

Le processus de purification, illustré sur la figure 2, commence par la dissolution de 10kg de 20E pure à 90%, dans 40L de méthanol, dans un premier réacteur.

La solution obtenue est ensuite chauffée à 45-50°C, jusqu'à apparition d'une coloration marron claire. La solution est alors filtrée à travers un filtre à particules de 0,2 µm dans un second réacteur. Le filtre à particules est rincé avec 5L de MeOH, dans ledit second réacteur, puis la solution est concentrée à un volume de 25L par distillation sous vide à une température de 50°C.

On ajoute 80L d'acétone comme anti-solvant à 45-50°C. On provoque la cristallisation de la 20E en refroidissant à 15-20°C et en agitant pendant 16 à 18 heures à 15-25°C, puis en refroidissant à 0-5°C et en agitant pendant au moins 4 heures. On filtre le précipité puis on le lave avec 20L d'acétone préalablement refroidie à 0-5°C, et on le sèche dans un courant d'azote gazeux pendant au moins 1,5 heure.

On charge le second réacteur avec le produit ainsi obtenu, qui est remis en suspension dans 20L d'eau pour éliminer l'acétone résiduelle. On agite la suspension à 15-25°C pendant 2 heures, puis on la filtre à travers le filtre à particules et on lave le matériau avec 20L d'eau purifiée avant de le sécher sous vide à une température inférieure ou égale à 50°C.

### 2. Analyse du produit fini

Le produit fini (BIO101) a été analysé par HPLC-MS/MS. Les composants mineurs ont été isolés par HPLC à partir des liqueurs-mères puis analysés en spectrométrie de masses et spectrométrie RMN. Il s'avère que les composants significatifs présents dans une quantité supérieure ou égale à 0,05% en poids sec de BIO101, sont exclusivement des phytoecdysones. Les valeurs reportées dans le tableau 1 ci-dessous correspondent à des moyennes sur quatre lots distincts d'extrait brut de racines (*Stemmacantha carthamoides*) et quatre lots distincts de produit fini. En ce qui concerne les données de l'extrait brut de racines, les valeurs sont rapportées à la somme des phytoecdysones observées en HPLC, mais en fait la 20E ne représente que 15-21% en poids dans le résidu séché de l'extrait alcoolique des racines séchées (n = 2).

**Tableau 1**

| Nom | Extraits de *Stemmacantha carthamoides* | | |
|---|---|---|---|
| | Extrait brut de racines | Matière Initiale (20E pure à 90%) | Produit fini (BIO101) |
| 20E | 60,04 | 93,57 | 97,10 |
| 3-Acétate de 20E | 16,47 | 0,86 | 0,27 |
| 2-Acétate de 20E | 7,7 | 0,94 | 0,32 |
| Ajugastérone C | 5,3 | 1,84 | 0,52 |
| Rubrostérone | 4,5 | 0,14 | - |
| Dihydrorubrostérone (17β-OH) | 0,71 | 0,04 | - |
| Poststérone | 0,67 | 0,07 | - |
| Dihydrorubrostérone (17α-OH) | 0,63 | - | - |
| 20,26-Dihydroxyecdysone (25R et 25S) | 0,54 | 0,81 | 0,68 |
| Turkestérone | 0,38 | 0,03 | 0,01 |
| 2-Désoxy-20,26-hydroxyecdysone | 0,37 | 0,27 | 0,18 |
| Inokostérone | 0,27 | 0,30 | 0,32 |
| 14-Désoxy-20-Hydroxyecdysone | 0,19 | 0,24 | 0,17 |
| Stachvstérone B | - | 0,16 | 0,06 |
| Autres phytoecdysones | 2,23 | 0,73 | 0,16 |

On notera qu'à l'issue de l'ensemble des étapes du procédé de purification, certaines phytoecdysones ont été éliminées. De plus, un composé nouveau est apparu : la stachystérone B. Ce dernier correspond à un produit de déshydratation issu de la perte de l'hydroxyle tertiaire en position 14 de la 20E. Ce composé est en partie éliminé lors de la purification, et aucun autre produit nouveau n'apparaît lors du procédé.

En ce qui concerne les quatre molécules éliminées, il s'agit de molécules issues d'une coupure de la chaîne latérale de la 20E et qui sont donc des molécules à 21C (poststérone) ou 19C (rubrostérone, dihydrorubrostérone). A la différence de la 20E, ces molécules sont susceptibles d'interagir avec les récepteurs nucléaires des androgènes ou des oestrogènes (Lapenna et al., 2015) et il est donc primordial de les éliminer afin d'éviter tout risque d'interférence voire d'effets indésirables du produit fini. Ce point est d'autant plus important que ces molécules, de polarité réduite par rapport à la 20E, ont une biodisponibilité très supérieure à cette dernière et sont donc proportionnellement beaucoup mieux (de l'ordre de vingt fois mieux) biodisponibles.

### 3. Caractérisation de la BIO101

Le tableau 2 ci-dessous présente la caractérisation de l'extrait obtenu ci-dessus.

**Tableau 2**

| Attribut | Spécification | Méthode |
|---|---|---|
| Caractéristiques | | |
| Aspect | Poudre blanche | Visuelle |
| Solubilité | | |
| *Dans l'éthanol* | Très soluble | |
| *Dans l'eau pure* | Légèrement soluble | Ph. Eur. |
| *Dans le DMSO* | Très soluble | |

| Identification | | |
|---|---|---|
| 20-hydroxyecdysone (temps de rétention en HPLC) | Temps de rétention identique à celui d'un composé de référence | HPLC |
| 20-hydroxyecdysone (spectre IR) | Identique à référence | Ph. Eur. 2.2.24 |

| Tests | | |
|---|---|---|
| Aspect en solution | | Ph. Eur. 2.2.1 |
| *Limpidité* | Solution transparente | Ph. Eur. 2.2.2 |
| *Coloration* | Pas plus colorée que YB5 | Method Il |
| Teneur en eau (% w/w) | 0,5% | Ph. Eur. 2.5.12 |
| Cendres sulfuriques | < 0,1 % | Ph. Eur. 2.4.14 |
| Cendres totales | < 0,1 % | Ph. Eur. 2.4.16 |
| Solvants résiduels (HS-GC/FID) | | Ph. Eur. 2.4.24 |
| *Méthanol* | ≤ 3000 ppm | |
| *Ethanol* | ≤ 5000 ppm | |
| *Acétone* | ≤ 5000 ppm | |
| *Acétate d'éthyle* | ≤ 5000 ppm | |
| Dosage par HPLC des impuretés apparentées à la 20E (%) | Rapport (cf.Tableau 1) | HPLC |
| *Impuretés inconnues* | | |
| *Impuretés totales* | < 3,5 % | |
| Impuretés minérales | | Ph. Eur. 2.4.8 |
| Cd, Pb | ≤ 0.5 µg/g | |
| As | ≤ 1.5 µg/g | |
| Hg | ≤ 3 µg/g | |
| Co | ≤ 5 µg/g | |
| Vd | ≤ 10 µg/g | |
| Ni | ≤ 20 µg/g | |
| Tl | ≤ 0.8 µg/g | |
| Au, Pd, Ir, Os, Rh, Ru | ≤ 10 µg/g | |
| Se, Ag | ≤ 15 µg/g | |
| Pt | ≤ 10 µg/g | |
| Li | ≤ 55 µg/g | |
| Sb | ≤ 120 µg/g | |
| Ba | ≤ 140 µg/g | |
| Mo, Cu | ≤ 300 µg/g | |
| Sn | ≤ 600 µg/g | |
| Polymorphisme (XRPD) | Forme cristalline anhydre | XRPD |

| Contamination Microbienne | | |
|---|---|---|
| TAMC | ≤ 10³ CFU/g | Ph. Eur. 5.14 |
| TYMC | ≤ 10² CFU/g | |
| *Escherichia coli* | Absence / 1g | |

| Dosage | | |
|---|---|---|
| Dosage de la 20-hydroxyecdysone par HPLC (% w/w, base anhydre sans solvant) | ≥ 95% | HPLC |

### 4. Stabilité de la BIO101

Le suivi de la stabilité de la BIO101 stocké à 25°C et 60% d'humidité relative révèle l'absence d'apparition d'impuretés au bout d'un an.

Le même résultat est obtenu pour de la BIO101 stocké à 30°C et 65% d'humidité relative.

Le produit obtenu s'avère donc très stable, il ne se dégrade pas et ne génère pas d'impuretés indésirables.

Des tests de dégradation forcée, sur des échantillons de BIO101 à 40°C et 75 % d'humidité relative pendant 6 mois, confirment l'excellent comportement de la BIO101, précisément du fait de sa très forte teneur en principe actif par rapport à la quantité de composés mineurs.

### 5. Eléments de toxicologie réglementaire

Des travaux réalisés avec des préparations de 20E partiellement purifiée provenant de *Pfaffia glomerata* se sont avérées génotoxiques et cytotoxiques (Neves et al., 2016a,b). Ceci est dû à la présence d'autres composés présents dans des extraits alcooliques de la plante (De Souza et al., 2005) et incomplètement éliminés dans la préparation de 20E utilisée.

En termes de génotoxicité, les résultats obtenus en suivant les règles ICH et OCDE en vigueur *in vitro* et *in vivo,* sur BIO101, ont été négatifs. Chez l'animal, aucune toxicité n'a été observée à la plus haute dose testée, soit 1000 et 1500 mg/kg respectivement chez le rat et le chien exposés de manière chronique pendant 28 jours. Ainsi, la 20E purifiée obtenue selon le procédé décrit plus haut (BIO101) n'est pas toxique, même à forte dose. La batterie de tests de « safety pharmaco » (comportement, SNC, fonction respiratoire, test de hERG et télémétrie cardiaque) confirme ce point.

L'administration chronique de BIO101 par voie orale à l'occasion de deux études GLP chez le rat et chez le chien pendant 26 semaines a confirmé l'excellente tolérance du produit ainsi que son profil de sécurité.

### 6. Études pharmacocinétique chez l'Homme

Chez l'Homme, Simon & Koolman (1989), puis Brandt (2003) et enfin Bolduc et al. (2008) ont mesuré les cinétiques d'élimination urinaire de la 20E partiellement purifiée, après administration orale, respectivement aux doses de 0,2 mg/kg*jour, 10 mg/kg*jour et 434 mg/jour, soit en dose unique, soit pendant 5 jours. Ces travaux mettent en évidence une faible biodisponibilité orale de cette molécule.

Les études réalisées chez l'animal et l'Homme avec BIO101 confirment la faible biodisponibilité de cette molécule après administration par voie orale. Ils montrent que la molécule ingérée est totalement éliminée après 48 heures, et ce principalement par voie fécale.

Dans le cadre d'une étude clinique de phase I, les concentrations plasmatiques de BIO101 ont été mesurées suite à des administrations uniques (SAD) de 100, 350, 700 et 1400 mg/jour chez des individus à jeun. Des administrations répétées (MAD) pendant 14 jours, ont été ensuite effectuées avec une dose journalière unique de 350 mg ou avec deux prises journalières (matin et soir) de 350 mg et 450 mg. Ces administrations de BIO101 à différentes cohortes de volontaires (n=6) ont permis de déterminer les principaux paramètres pharmacocinétiques de BIO101 : Cₘₐₓ (concentration plasmatique maximale de BIO101 après unique ou répétée), Cₘᵢₙ (concentration plasmatique minimale de BIO101 après administration unique ou répétée), Tₘₐₓ (temps nécessaire pour atteindre la concentration plasmatique maximale en BIO101 après administration unique ou répétée), et AUC (Aire sous la courbe) synonyme du niveau d'exposition plasmatique. Ces résultats sont présentés dans le tableau 3 ci-dessous, et les valeurs correspondent aux moyennes arithmétiques (CV%) sauf pour Tₘₐₓ où ce sont les valeurs extrêmes.

**Tableau 3**

| Paramètre mesuré | Dose administrée | | | |
|---|---|---|---|---|
| | 100 mg | 350 mg | 700 mg | 1400 mg |
| Cₘₐₓ (ng/mL) | 141 (16,6) | 317 (37,9) | 399 (24,7) | 710 (20,2) |
| tmax (h) | 2,03 (1,00-3,02) | 3,00 (1,05-4,00) | 3,00 (2,00-4,02) | 3,50 (2.,00-4,02) |
| AUC₀₋ₜ (ng.h/mL) | 767 (31,1) | 1924 (40,1) | 2578 (22,9) | 4148 (15,9) |
| AUC_{0-∞} (ng.h/mL) | 797 (32,8) | 1946 (39,4) | 2658 (20,4) | 4283 (17,4) ⁿ⁼⁵ |
| t_{1/2} (h) | 2,40 (29,0) | 3,26 (43,5) | 4,85 (66,9) | 3.84 (24,1) ⁿ⁼⁵ |
| CLr (L/h) | 4,23 (22,0) | 4,05 (17,3) | 4,43 (36,1) | 5.05 (18,6) |
| Cₘₐₓ/dose (ng/mL/mg) | 1,41 (16,6) | 0,905 (37,9) | 0,570 (24,7) | 0.507 (20,2) |
| AUC₀₋ₜ/dose (ng.h/mL/mg) | 7,67 (31,1) | 5,50 (40,1) | 3,68 (22,9) | 2.96 (15,9) |
| AUC_{0-∞}/dose (ng.h/mL/mg) | 7,97 (32,8) | 5,56 (39,4) | 3,80 (20,4) | 3.06 (17,4) ⁿ⁼⁵ |
| Ae₀₋₂₄ₕ (% dose) | 3,41 (35,2) | 2,22 (41,4) | 1,61 (41,3) | 1,51 (35,9) |

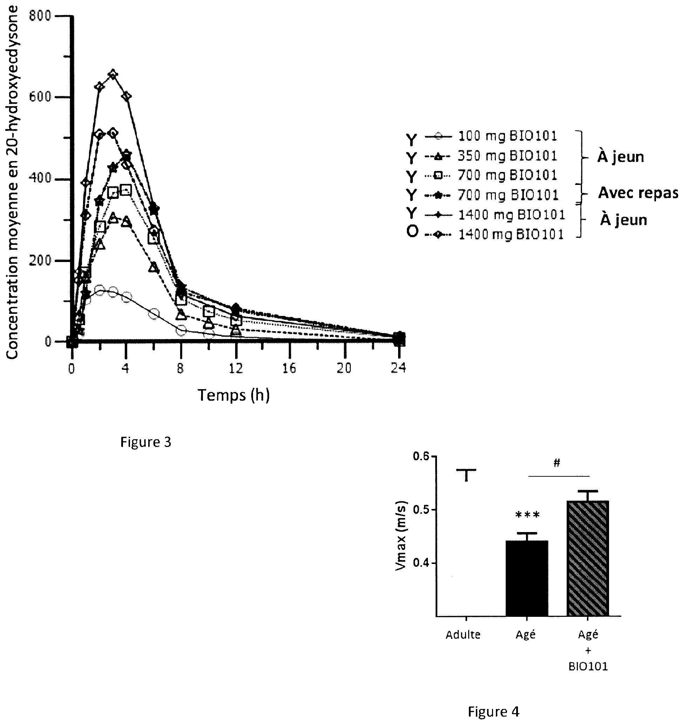

Le profil moyen des cohortes de la SAD est présenté dans la figure 3 où Y sont des volontaires jeunes et O sont des volontaires dont l'âge est compris entre 65 et 85 ans. Cette figure montre un Tₘₐₓ entre 2 et 3,5 heures après l'ingestion, un Cₘₐₓ qui augmente avec la dose ingérée, tout en restant faible dans tous les cas, en accord avec la faible biodisponibilité de la 20E et la rapide élimination de l'actif, qui disparaît presque totalement après 24 heures. La demi-vie de la 20E est de l'ordre de 3-4 heures.

Le tableau 4, ci-dessous, illustre les paramètres pharmacocinétique suite à des administrations répétées de BIO101. Ce tableau montre que les administrations répétées présentent des profils très similaires entre le premier et le quatorzième jour, en accord avec une absence d'accumulation plasmatique du composé ingéré ainsi qu'avec l'absence d'induction de mécanismes de détoxication avec les doses utilisées.

**Tableau 4**

| Paramètres (Unités) | 350 mg 1X | | 350 mq 2X | | 450 mg 2X | |
|---|---|---|---|---|---|---|
| | J1 | J 14 | J1 | J 14 (N=7)^{¥} | J1 | J 14 |
| Cₘₐₓ (ng/mL) | 346 (1,6) | 388 (23,5) | 453 (29,9) | 506 (15,7) | 524 (32,9) | 560 (34,5) |
| tmax (h) | 3,00 (2,00-4,00) | 3,00 (3,00-4,00) | 3,00 (2,02-4,37) | 3,00 (2,00-4,00) | 3,00 (3,00-3,00) | 3,00 (2,00-4,00) |
| AUC₀₋ₜ (ng.h/mL) | 2140 (18,4) | 2389 (18,0) | 2230 (17,6) | 2766 (14,5) | 2424 (28,5) | 3203 (37,9) |
| AUC_{0-τ} (ng.h/mL) | 2141 (18,4) | 2389 (18,0) | 2234 (17,6) | 2768 (14,5) | 2429 (28,5) | 3203 (37,8) |
| AUC_{0-∞} (ng.h/mL) | 2193 (18,0) | 2414 (18,0) | 2451 (16,0) | 3028 (15,0) | 2566 (28,1) n=7 | 3486 (38,6) |
| t_{1/2} (h) | 4,39 (20,7) | 3,39 (10,9) | 3,00 (12,1) | 3,06 (21,3) | 3,72 (28,1) | 2,81 (8,62) |
| CLr (L/h) | 4,08 (23,1) | 3,84 (14,8) | 3,46 (14,3) | 4,00 (12,7) | 3,57 (13,8) | 4,41 (21,1) |
| Rac | NA | 1,14 (23,6) | NA | 1,31 (16,7) | NA | 1,31 (20,2) |
| Ae₀₋₁₂ₕ (%dose) | 2,17 (34,0) | 2,38 (25,6) | 2,21 (24,8) | 3,14 (13,2) | 1,88 (20,3) | 3,01 (31,1) |
| Cₘₐₓ/dose (ng/mL/mg) | 0,988 (16,6) | 1,11 (23,5) | 1,29 (29,9) | 1,45 (15,7) | 1,16 (32,9) | 1,24 (34,5) |
| AUC_{0-τ}/dose (ng,h/mL/mg) | 6,12 (18,4) | 6,83 (18,0) | 6,38 (17,6) | 7,91 (14,5) | 5,40 (28,5) | 7,12 (37,8) |

Les valeurs correspondent aux moyennes arithmétiques (CV%) sauf pour Tₘₐₓ où se sont les valeurs extrêmes. N=8 sauf indication contraire; n=nombre de sujets pour cette observation; NA= ne s'applique pas. * Un sujet est sorti de l'étude après 10 jours.

Ces résultats sont compatibles avec une utilisation chronique de BIO101, qui a été également validée par l'absence de toxicité suite à une administration quotidienne pendant 6 mois à des doses de 1000 mg/kg*j chez le rat et 1500 mg/kg*j chez le chien.

### 7. Activité biologique de BIO101

Si les effets anabolisants de la 20E présente dans des préparations commerciales ont déjà été démontrés chez des animaux jeunes, on ne sait pas s'il en est de même dans un contexte âgé, qui s'accompagne d'une baisse de la masse des muscles (sarcopénie) et surtout de leurs performances (dynapénie), qui provoquent des problèmes de mobilité. BIO101 a été administré de manière chronique à des souris âgées et la vitesse maximale de course des animaux a été mesurée et comparée à celle d'animaux âgés et adultes non traités. Cette étude a démontré que les effets anaboliques/hypertrophiques de BIO101 se traduisent par une amélioration fonctionnelle caractérisée par une augmentation de la vitesse maximale de course.

Des souris femelles C57BI6/J adultes (12 mois) et âgées (22 mois) ont été utilisées. Un groupe de souris âgées a reçu BIO101 par voie orale pendant 13 semaines à la dose de 50 mg/kg*jour et un autre groupe le véhicule. Les souris adultes ont été exposées au véhicule. Tous les animaux ont été exposés à un régime hypercalorique riche en lipides pendant toute la durée de l'expérimentation (4442 kcal/kg ; protéines 19.8%, lipides 23% et carbohydrates 39.5%). Après 13 semaines de traitement, les animaux ont été testés pour leur capacité fonctionnelle (activité *in toto*)*.* Ils ont été soumis à un exercice de course et la vitesse maximale de course a été enregistrée.

Les résultats sont illustrés sur la figure 4. De manière attendue, on constate que les animaux âgés non traités courent significativement plus lentement (-20%, p<0.001) que les animaux adultes non traités. Les animaux âgés qui ont reçu BIO101 courent quant à eux significativement plus vite (+17%, p<0.05) que les animaux âgés contrôles (véhicule).

De manière importante, nous démontrons ici que le traitement des animaux âgés par BIO101 compense pour une grande part la perte significative de vitesse de course due au vieillissement des animaux. Il existe en effet un important recouvrement des intervalles de confiance 95% des groupes *âgé + BIO101* (95% Cl = 0.471 à 0.558 m/s) et *Adulte non traité* (95% Cl = 0.503 à 0.602 m/s). Le traitement par BIO101 permet ainsi de limiter la perte fonctionnelle liée au vieillissement à 7%, au lieu des 20% observés chez les animaux âgés non traités.

Ces travaux démontrent que les propriétés bénéfiques de l'actif de BIO101 sur la fonction musculaire (Chermnykh et al., 1988 ; Gorelick-Feldman et al., 2008 ; Toth et al., 2008) s'appliquent dans le contexte du mammifère âgé, et que le traitement chronique d'animaux âgés par BIO101 peut compenser significativement les pertes fonctionnelles dues au vieillissement.

Plus largement, et compte tenu des propriétés de BIO101 sur la fonction musculaire, l'utilisation de BIO101 peut donc être proposée pour traiter des affections qui ont pour conséquence la détérioration de ladite fonction musculaire. Ces affections incluent, mais ne sont pas limitées à la sarcopénie, les dystrophies musculaires, notamment d'origine génétique et la perte de masse et de fonction musculaires suite à une immobilisation, notamment dans un contexte post chirurgical.

### LISTE DES RÉFÉRENCES

Bathori M, Girault J-P, Kalasz, H, Mathé I, Dinan LN, Lafont R. 1999. Complex phytoecdysteroid cocktail of Silene otites (Caryophyllaceae). Arch. Insect Biochem. Physiol. 41(1): 1-8.
Bolduc TM. 2008. Human urinary excretion profiles after exposure to ecdysterone. Master Thesis, University of Utah.
Brandt FW. 2003. Pharmakokinetik und Metabolismus des 20-Hydroxyecdysons im Menschen. PhD Thesis, University of Marburg.
Chermnykh NS, Shimanovsky NL, Shutko GV, Syrov VN. 1988. Effects of methandrostenolone and ecdysterone on physical endurance of animals and protein metabolism in the skeletal muscles. Farmakologiya i Toksikologiya 6 : 57-62. Gorelick-Feldman J, MacLean D, Ilic N, Poulev A, Lila MA, Raskin I. 2008. Phytoecdysteroids increase protein synthesis in skeletal muscle cells. J. Agric. Food Chem. 56: 3532-3537.
Kokoska L, Janovska D. 2009. Chemistry and pharmacology of Rhaponticum carthamoides: a review. Phytochemistry 70: 842-855.
Lapenna S, Gemen R, Wollgast J, Worth A, Maragkoudakis P, Caldeira S. 2015. Assessing herbal products with health claims. Critical Reviews in Food Science & Nutrition 55(13): 1918-1928.
Simon P, Koolman J. 1989. Ecdysteroids in vertebrates : pharmacological aspects. In « Ecdysone - from chemistry to mode of action », J. Koolman Ed., Georg Thieme Verlag, Stuttgart, pp. 254-259.
Toth N, Bathori M. 2008. Preparative-Scale Chromatography of Ecdysteroids: A Class of Biologically Active Steroids. Journal of Chromatographie Science, 46(2), 111-116. Tóth N, Szabó A, Kacsala P, Héger J, Zádor E. 2008. 20-Hydroxyecdysone increases fiber size in a muscle-specific fashion in rat. Phytomedicine 15: 691-698.

### ANNEXE

### Exemple de certificat d'analyse de la matière première

| | |
|---|---|
| **Nom du produit :** | *Leuza carthamoides* Powder Extract |
| **Nom latin :** | *Leuzea carthamoides* (Wild.) DC |
| **Partie de plante utilisée :** | Feuilles / racines |
| **Méthode d'extraction :** | Extraction à l'eau et à l'éthanol |

| **Item** | **Specification** | | **Result** |
|---|---|---|---|
| **Physical Items:** | | | |
| Appearance: | Light Yellow fine powder | | Conformed |
| Taste and Odor: | Characteristic | | Characteristic |
| Mesh Size | 100% through 80 Mesh (USP34<786>) | | Conformed |
| Identification | TLC | | Conformed |

| **Chemical Items:** | | | |
|---|---|---|---|
| Extract Ratio/Active Ingredients: | Beta ecdysterone 90% Min HPLC | | 91.67% |
| Loss on Drying | 5.0% Max (Eur.Ph.6.0<2.8.17>) | | 4.10% |
| Sulphated Ash | 1.0% Max (Eur.Ph.7.0<2.4.16>) | | 0.20% |
| Residual Solvent | 50 ppm MaxUSP34 <467> | | Conformed |
| Residual Pesticides | 10ppb Max (PCNB,DDT,BHC) | | Conformed |
| Heavy Metals | 10 ppm Max (CP2010) | | Conformed |
| Arsenic (As) | 2 ppm Max (ICP-MS) | | Conformed |
| Lead (Pb) | 2 ppm Max (ICP-MS) | | Conformed |
| Cadmium (Cd) | 1 ppm Max (ICP-MS) | | Conformed |
| Mercury (Hg) | 0.1 ppm Max (ICP-MS) | | Conformed |
| GMO Status | GMO Free | | Conformed |
| BSE/TSE Status | BSE/TSE Free | | Conformed |
| Preservative status | Preservative Free | | Conformed |
| Irradiated Status | Non-irradiated | | Conformed |
| Pesticides | Negative | | Conformed |
| Carrier Used or Excipients Added | None | | Conformed |
| **Sterilization method** | High Température & Pressure | | Conformed |

| **Microbiology Control** | | | |
|---|---|---|---|
| Total Plate Count | 10,000 cfu/g (USP) | | Conformed |
| Total Yeast & Mold | 1,000 cfu/g (USP) | | Conformed |
| E.Coli | Negative (USP) | | Conformed |
| Salmonella | Negative (USP) | | Conformed |
| Staphylococcus | Négative (USP) | | Conformed |
| | | | |

| **The product could be used for human consumption.** | | | |
|---|---|---|---|
| Packaging: | | 25kg/Drum | |
| Storage | | Store in a cool dry area. Do not freeze. Keep away from stronq direct light. | |
| Shelf Life | | 2 Years when properly stored. | |

## Revendications

1. Procédé de préparation d'un extrait comportant au moins 95%, de préférence au moins 97%, de 20-hydroxyecdysone, à partir d'une préparation de 20-hydroxyecdysone pure de l'ordre de 90%, **caractérisé en ce qu'**il comprend les étapes suivantes :
a) Dissolution à chaud de 20-hydroxyecdysone pure à de l'ordre de 90% dans du méthanol, filtration et concentration partielle,
b) Ajout de 1 à 5 volumes d'acétone,
c) Refroidissement à une température comprise entre 0 et 5°C sous agitation,
d) Filtration du précipité obtenu,
e) Rinçages successifs avec de l'acétone et de l'eau, et
f) Séchage.

2. Procédé selon la revendication précédente, **caractérisé en ce que** la filtration de l'étape a) est réalisée grâce à un filtre à particules de 0,2 µm, **en ce que** la concentration partielle de l'étape a) est effectuée par distillation sous vide, à une température de l'ordre de 50°C, en présence de MeOH, et **en ce que** l'étape f) de séchage est effectuée sous vide à une température de l'ordre de 50°C.

## Patentansprüche

1. Verfahren zur Herstellung eines Extrakts, das mindestens 95 %, vorzugsweise mindestens 97 % 20-Hydroxyecdyson enthält, aus einer Zubereitung von reinem 20-Hydroxyecdyson in der Größenordnung von 90 %, **dadurch gekennzeichnet, dass** es die folgenden Schritte umfasst:
a) Auflösung unter Hitze von etwa 90 % reinem 20-Hydroxyecdyson in Methanol, Filtration und teilweise Konzentration,
b) Zugabe von 1 bis 5 Volumenteilen Aceton,
c) Abkühlung auf eine Temperatur von 0 bis 5 °C unter Rühren,
d) Filtration des erhaltenen Niederschlags,
e) Aufeinanderfolgende Spülungen mit Aceton und Wasser und
f) Trocknung.

2. Verfahren nach dem vorhergehenden Anspruch, **dadurch gekennzeichnet, dass** die Filtration aus Schritt a) durch einen Partikelfilter von 0,2 µm erfolgt, dadurch, dass die teilweise Konzentration aus Schritt a) durch Destillation unter Vakuum bei einer Temperatur in der Größenordnung von 50 °C in Gegenwart von MeOH durchgeführt wird, und dadurch, dass der Schritt f) der Trocknung unter Vakuum bei einer Temperatur in der Größenordnung von 50 °C durchgeführt wird.

## Claims

1. Method for preparing an extract including at least 95%, preferably at least 97%, of 20-hydroxyecdysone, from a preparation of approximately 90% pure 20-hydroxyecdysone, **characterised in that** it comprises the following steps:
a) Hot dissolution of approximately 90% pure 20-hydroxyecdysone in methanol, filtration and partial concentration,
b) Addition of 1 to 5 volumes of acetone,
c) Cooling to a temperature of between 0 and 5°C while stirring,
d) Filtration of the obtained precipitate,
e) Successive rinses with acetone and water, and
f) Drying.

2. Method according to the previous claim, **characterised in that** the filtration of step a) is carried out via a 0.2µm particle filter, **in that** the partial concentration of step a) is performed by vacuum distillation, at a temperature of approximately 50°C, in the presence of MeOH, and **in that** step f) of drying is performed under vacuum at a temperature of approximately 50°C.
